(19) Europäisches Patentamt
European Patent Office
Office européen des brevets

(11) Publication number: **0 242 656**
**A2**


# (12) EUROPEAN PATENT APPLICATION


(21) Application number: 87104853.4

(22) Date of filing: 02.04.87

(51) Int. Cl.³: C 12 P 21/00 //C12N9/02

(30) Priority: 25.04.86 US 856594

(43) Date of publication of application: 28.10.87 Bulletin 87/44

(84) Designated Contracting States: AT BE CH DE ES FR GB GR IT LI LU NL SE



(71) Applicant: Bio-Polymers, Inc.
309 Farmington Avenue
Farmington Connecticut 06032(US)

(72) Inventor: Benedict, Christine V.
36 Gail Road
Farmington, Connecticut 06032(US)

(72) Inventor: Picciano, Paul T.
31 Colony Road
Canton, Connecticut 06109(US)

(74) Representative: Hoeger, Stellrecht & Partner
Uhlandstrasse 14c
D-7000 Stuttgart 1(DE)


## (54) Method for making dopa-containing bioadhesive proteins from tyroshine-containing proteins.


(57) Methods of forming bioadhesive polyphenolic proteins containing 3,4-dihydroxyphenylalanine residues from protein precursors containing tyrosine residues are provided comprising:

preparing a tyrosine-containing protein;

reacting said protein with a tyrosinase enzyme at a pH of from about 4.5 to about 8 and a temperature of from about 20° to about 37°C. at an enzyme to protein ratio of from about 5 to about 50 Units enzyme per µg protein; and thereby forming a bioadhesive polyphenolic protein containing 3,4-dihydroxyphenylalanine residues.


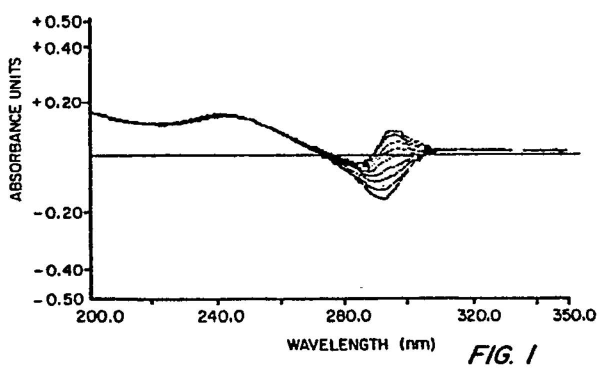

*FIG. 1*


EP 0 242 656 A2

# METHOD FOR MAKING DOPA-CONTAINING BIOADHESIVE PROTEINS FROM TYROSINE-CONTAINING PROTEINS

## BACKGROUND OF THE INVENTION

### Field of the Invention

This invention relates to the production of bioadhesive polyphenolic proteins containing dihydroxyphenylalanine (L-dopa) residues from precursors containing tyrosine-containing residues. The bioadhesive polyphenolic proteins produced exhibit unusually superior adhesive capabilities toward a variety of surfaces, including surfaces exposed to high levels of humidity or surfaces submerged under water, whereas the tyrosine-containing precursors do not exhibit those properties.

### Description of the Prior Art

Bioadhesive polyphenolic proteins derived from natural biological sources are the basis for a group of bioadhesives which cure underwater or in high humidity environments, and remain flexible and extensible after curing. These bioadhesives are resistant to corrosion and biological attack to varying degrees, dependent on precise composition and cure conditions.

Bioadhesive polyphenolic proteins, originally derived from several species of the mussel genus *Mytilus*, can be derived either from natural sources or manufactured synthetically, and contain one or more sequences of repeating decapeptides having the formula:

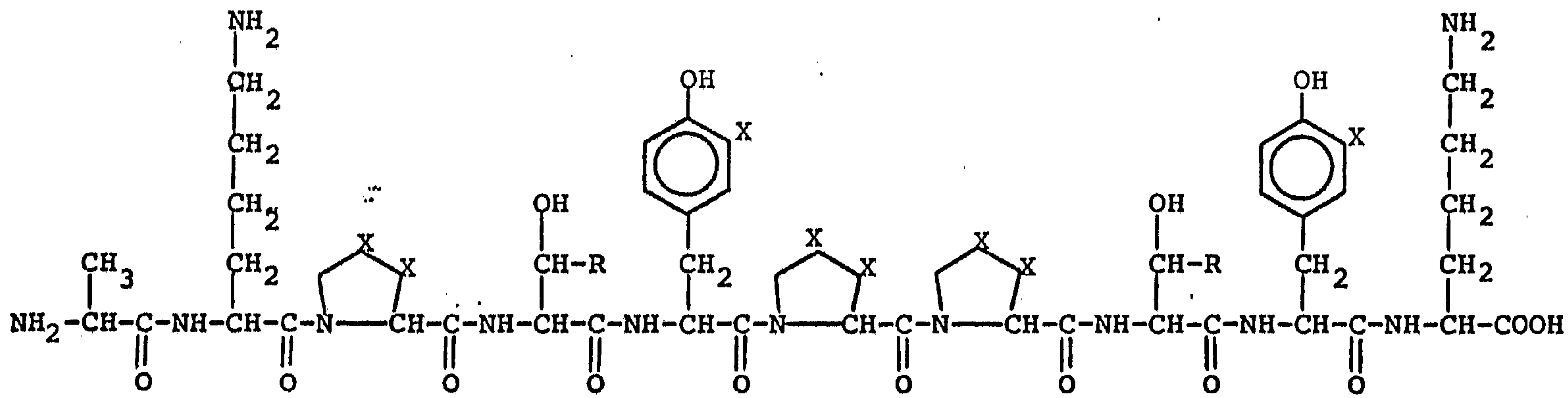

ALA LYS PRO/HYP SER/THR TYR/DOPA PRO/HYP PRO/HYP SER/THR TYR/DOPA LYS

wherein each X is independently selected from the group consisting of hydroxyl and hydrogen; and wherein each R is independently selected from the group consisting of hydrogen and methyl. As used in this application, the term "bioadhesive polyphenolic proteins" is to be understood as referring to mixtures of proteins containing from 1 to about 1,000 units of the above repeating decapeptides, and optionally may contain other proteinaceous units and chain linkers.

Most known adhesives interact well with a clean surface containing a monolayer of water, such water providing sites for hydrogen bonding with the adhesive. For known adhesives, however, any additional water will weaken the interaction at the surface or hydrolyze or plasticize the adhesive. For bioadhesive polyphenolic protein adhesives, on the other hand, the amine, hydroxyl, and catechol residues together provide the structure with an overwhelming number of reactive sites capable of all levels of interactions (covalent, ionic, hydrogen, and Van der Waals bonding) for cohesive and adhesive strength. The molecular weight of the bioadhesive proteins (130,000) enhances cohesive strength while the bulky, polar side chains prevent crystal formation and promote molecular entanglements with the substrate.

The repeating decapeptide structure of the bioadhesive polyphenolic protein derived from the marine mussel is described in the United States Patent No. 4,585,585 entitled "Decapeptides Produced from Bioadhesive Polyphenolic Proteins". Methods of extracting the bioadhesive polyphenolic protein from Mytilus are known in the art (Waite and Tanzer, 1981, Science 212, 1038) as are methods for the digestion of such extracted proteins into repeating peptides (Waite 1983, J. Biol. Chem 24, 5010). Heretofore, however, no method for the synthetic preparation of bioadhesive polyphenolic proteins has been known.

The synthesis of proteins by organic methods is complex. Currently, synthesizers are available which are semi- or fully automated and therefore simplify the process, at least on a small scale (i.e., up to 2.0 gms.). The L-dopa precursor required for this methodology is not commercially available and if synthesized properly and incorporated in the organic scheme, efficiency is in the range of 50-80%. It is possible to incorporate tyrosine in the direct synthesis scheme and use enzymatic modification of the finished tyrosine-containing protein to accomplish the incorporation of L-dopa. Monophenol monooxygenases (tyrosinases) have been described in yeasts, fungi, plants, and animals. This type of enzyme is generally known as the agent responsible for the quinone tanning reaction seen in mushrooms, fruits and insects. Mammalian tyrosinases have

been described in melanocytes, cells responsible for melanine formation (Jimbow, et al, J. Invest. Dermatol. 75, 122-127, 1976). The mechanism is thought to involve tyrosinase conversion of tyrosine in proteins to dopa which then crosslinks to other proteins through available cystine residues (Ito, et al, Biochem J. 222, 407-411, 1984). Other proteins which have been modified by tyrosinase include insulin (Cory and Frieden, Biochemistry, 6, 116-120, 1967), yeast alcohol dehydrogenase (Cory and Frieden, Biochemistry 6, 121-126) and bovine serum albumin (Frieden, et al., Fed. Proc. Fed. Am. Soc. Exp. Biol. 18, 438, 1959). All these proteins were converted with tyrosinase to S-S-cysteinyl dopa crosslinked structures and analyzed by Ito, et al. (Biochem. J. 222, 407-411, 1984).

## SUMMARY OF THE INVENTION

Accordingly, it is the primary object of the present invention to provide methods for the synthesis and use of bioadhesive polyphenolic proteins from tyrosine-containing precursors.

This as well as other objects and advantages are accomplished by the present invention which provides a method of forming bioadhesive polyphenolic proteins containing 3,4-dihydroxyphenylalanine residues from protein precursors containing tyrosine residues comprising:

preparing a tyrosine-containing protein;

reacting said protein with a tyrosinase enzyme at a pH of from about 4.5 to about 8 and a temperature of from about 20° to about 37°C. at an enzyme to protein ratio of from about 5 to about 50 Units enzyme per μ g protein; and,

thereby forming a bioadhesive polyphenolic protein containing 3,4-dihydroxyphenylalanine residues.

The present invention will be more readily understood by reference to the drawings wherein;

Figure 1 is a graphical representation of relative absorbance versus wave length (nm) for the conversion of tyrosine-containing peptide to L-dopa-containing peptide in accordance with Example 1.

Figure 2a is a graphical representation of absorbance at 280 nm versus time in minutes of the parent decapeptide employed in Example 1;

Figure 2b is a graphical representation of absorbance at 280 nm versus time in minutes of the products obtained in accordance with Example 1;

Figure 3 is a graphical representation of the concentration of L-dopa ( μ g/ml) versus time in minutes obtained in accordance with Examples 7-8; and

Figure 4 is a graphical representation of the breaking force (gm/1.3 $cm^2$) versus the ratio of enzyme to bond protein (Units enzyme/ μ g bond protein) in accordance with Example 9.

DETAILED DESCRIPTION OF THE INVENTION

A linear polymer precursor to the bioadhesive polyphenolic proteins may be fully synthesized using traditional peptide methods utilizing appropriately blocked and protected amino acids. Alternatively, the linear polymer precursor may comprise a primary sequence similar to that of the bioadhesive polyphenolic proteins synthesized genetically (i.e., the gene sequence translated using biochemical protein synthesis reagents by methods known in the art) exclusive of any hydroxylated prolines or tyrosines. In addition, the linear polymer precursor may be any combination of these two, and may contain other compatible polymers. Compatible polymers can be defined as amine-rich, hydroxyl-rich proteins or organic polymers which, when used in combination with bioadhesive polyphenolic proteins, do not adversely affect the adhesion/cohesion system.

One linear polymer precursor to the bioadhesive polyphenolic protein may be depicted as follows:

0242656

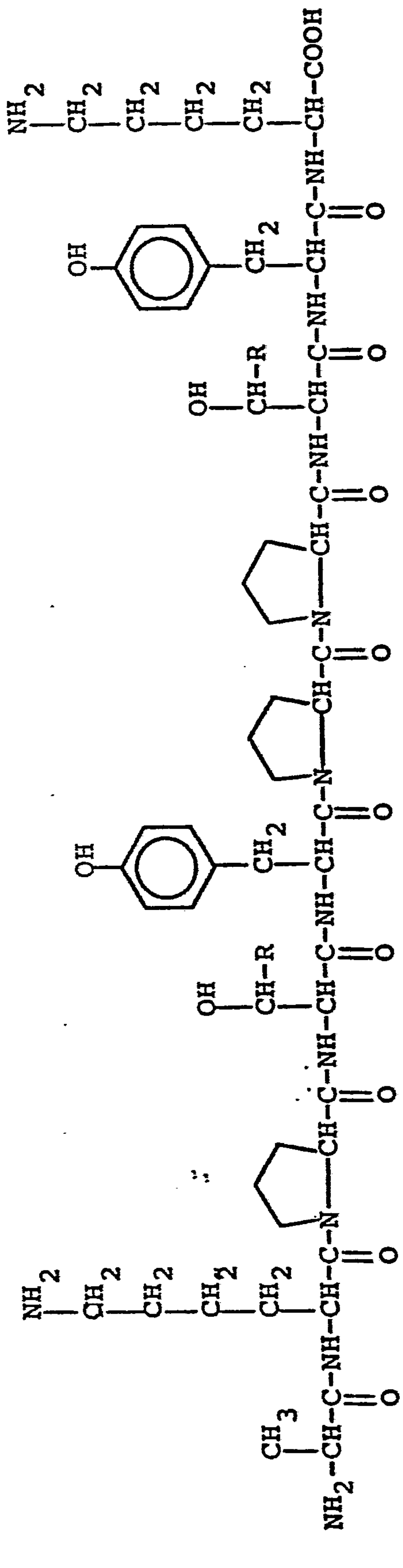

ALA LYS PRO SER/THR TYR PRO PRO SER/THR TYR LYS

wherein each R is independently selected from the group consisting of hydrogen and methyl.

In the case where the linear polymer precursor is synthesized without L-dopa or an L-dopa equivalent, tyrosine or a tyrosine equivalent can be converted chemically or enzymatically to the catecholic residue. Similarly, if proline is included in the linear polymer precursor, it can be enzymatically converted to the hydroxyproline residue.

The chosen sequence of the synthetic steps must consider several characteristics of the class of proteins: 1) the insolubility of extracted bioadhesive polyphenolic proteins at neutral pH or at low pH in the presence of salts (.5-3M NaCl, KCl, or LiCl) is a function of the high lysine, tyrosine and L-dopa; 2) short peptides with numbers of amino acid residues greater than about 20, with the same proportion of these amino acids, have similar solubility characteristics as do short peptides containing tyrosine but not L-dopa; 3) blocked, fully protected L-dopa is not commerically available and because of the extreme lability of this molecule, its synthesis and purification with subsequent incorporation into peptides is difficult at best; 4) with the incorporation of L-dopa, the peptide or protein becomes extremely sensitive to metals, oxidation, and loss due to binding to glassware and other proteins.

Several tyrosine-rich proteins and peptides have been enzymatically hydroxylated in accordance with the present

invention. These are:

1) synthetic proteins; the decapeptides described in the U.S. Patent No. 4,585,585 containing hydroxyproline but not L-dopa; tyrosine is present at 200 residues per thousand; the decapeptides used were single decapeptides and up to 5 repeats of the decapeptide;

2) extracted casein, a natural protein containing 46 residues per thousand tyrosine; and

3) extracted collagen, natural protein containing 10 residues per thousand tyrosine.

The following examples further illustrate specific embodiments of the present invention but are not to be construed as imposing any limitations on either the spirit or scope of the present invention.

## EXAMPLES 1 - 3

### The Conversion of Tyrosine Residues to L-Dopa Residues in Decapeptides

In accordance with the present invention, the enzymatic conversion of tyrosine residues to L-dopa residues can be mediated by the class of enzymes called tyrosinases: Monophenol monooxygenase; polyphenol oxidase; catechol

oxidase; monophenol, dihydroxyphenylalanine: oxygen oxidoreductase; (EC 1.14.18.1), and the like. In these examples, the readily available mushroom tyrosinase was used. These enzymes mediate not only the conversion of tyrosine to L-dopa, but the conversion of L-dopa to quinone as well. However, this latter reaction can be retarded or reversed in the presence of reductants such as L-ascorbic acid bisulfite, and the like.

The synthetic decapeptide is prepared by solid phase peptide synthesis. The term "solid phase" refers to the method by which synthesis occurs; that is, fixed to a matrix and the first amino acid is attached to a chromatographic resin. The linear chain of amino acids is produced by the sequential addition of each amino acid, one at a time, under conditions which promote formation of the amide bond. Labile side groups, such as the hydroxyls of serine, threonine and tyrosine, are added with protective or "blocking" groups which are deblocked after synthesis is complete. The peptides are cleaved from the matrix at the end of the final amino acid addition, de-blocked and purified.

In general, the conversion of tyrosine to L-dopa will occur in the pH range of 4.5 to 8, and preferably at pH 7 to 7.5. The reaction occurs rapidly at room temperature and above (20-37$^{o}$C) and very slowly at 4$^{o}$C. Three buffers tested were compatible: 0.1 M sodium phosphate, pH 7.0; 0.1 M sodium acetate, pH 5.0; and 0.1 M 2[N-morpholino] ethane sulfonic acid (pH 7.0). Adjustment of the enzyme-to-protein substrate ratio, temperature, and pH can be used to regulate the reaction rate. For most of the described examples, the enzyme-to-substrate ratio was 5.4 to 15 Units enzyme per μ g protein substrate. The pH was 7.0 in 0.1 M sodium phosphate buffer and the temperature was 21-23°C. L-ascorbic acid is included in the examples which require biochemical determination of reaction products because it retards and reverses the second reaction, the conversion of L-dopa to quinone. In the case of adhesive determination, the L-ascorbic acid is usually omitted to allow covalent cross-link formation. The addition of an equal volume of 10% v/v acetic acid to any conversion reaction rapidly decreases the pH to below 3.0 and terminates the reaction.

In Example 1, the reaction mixture, kept at room temperature, consists of 0.9 ml sodium phosphate buffer (0.1 M, pH 7.0), 0.1 ml synthetic single unit tyrosine-containing decapeptide (10 mg/ml in 0.1 M sodium phosphate buffer, pH 7.0) 0.025 ml tyrosinase (216 Units/μ l in 0.1 M sodium phosphate buffer and 0.01 ml L-ascorbic acid (0.1 M in the same phosphate buffer). As can be seen in Figure 1, over a ten minute time period, the wavelength of maximum change (decrease) is 292-294 nm. As a control run without L-ascorbic acid, the same reaction is complete in three minutes and quinone residue formation from L-dopa is rapid.

The reaction was characterized more precisely using a high pressure liquid chromatograph as the analytical tool,

together with amino acid composition analysis of the products. The reaction was slowed with the addition of more L-ascorbic acid (50 x the above concentration). The two product peaks are chromatographically distinct peaks and are believed to represent hydroxylation of tyrosine in the number 9 position or number 5 position, either one producing an L-dopa-containing peptide. The area of the parent decapeptide peak and product peaks were integrated as an indicator of percent conversion (see Figures 2a and 2b). In Example 2, a 7.0 pH sodium phosphate buffer was used as in Example 1. In Example 3, a 5.0 pH sodium acetate buffer was used instead of the phosphate buffer. The results are summarized in Table 1.

TABLE 1

A. Phosphate buffer pH 7.0 conversion

| | Percent Peptide | | |
|---|---|---|---|
| Time (hr) | Parent | Product 1 | Product 2 |
| 0 | 100 | | |
| 1 | 19 | 64 | 17 |
| 2 | 12 | 63 | 25 |

B. Sodium Acetate pH 5.0 conversion

| | | | |
|---|---|---|---|
| 0 | 100 | | |
| 1 | 40 | 60 | 0 |
| 2 | 26 | 68 | 6 |
| 4 | 20 | 72 | 8 |

EXAMPLES 4 - 6

Proteins containg 3, 4, and 5 repeats of decapeptides having tyrosine residues are converted in pH 7.0 sodium phosphate buffer at an enzyme-to-substrate ratio of 43 Units/$\mu$ g decapeptide. The enzyme-to-substrate ratio is increased here because the conversion of larger peptides is slower than for singlet decapeptides. Chromatographic analysis of the product distribution is not possible because the number of product peaks is increased proportionally to the number of tyrosines to be hydroxylated. Chromatography was used to verify that the reaction had gone to completion. The reaction mixture at room temperature (21-23$^{o}$C) in 0.1 M sodium phosphate buffer (pH 7.0) consisted of: 21,600 Units tyrosinase, 500 $\mu$ g synthetic peptide, and 100 $\mu$l of 1.0 M L-ascorbic acid in a total volume of 2.0 ml.

Table 2 provides the amino acid compositions of the reaction products. The enzyme and L-ascorbic acid were separated from the product by ultrafiltration.

## Table 2

### Amino Acid Composition (Key Amino Acids Only) of Tyrosinase Conversion Product from Synthetic Repeated Decapeptides

| | Unconverted decapeptide 1, 3, 5x | 3x product composite | 4x product composite | 5x product composite |
|---|---|---|---|---|
| 4-hydroxy-proline | 206 | 173 | 145 | 116 |
| threonine | 96 | 92 | 90 | 82 |
| serine | 86 | 102 | 98 | 107 |
| proline | 99 | 103 | 101 | 98 |
| alanine | 99 | 99 | 95 | 91 |
| L-dopa | - | 70 | 73 | 61 |
| tyrosine | 203 | 92 | 113 | 116 |
| lysine | 197 | 167 | 177 | 155 |

These amino acid compositions of the products, when compared to the starting material, confirm the conversion of tyrosine residues to L-dopa residues.

## EXAMPLES 7-8

Two natural proteins were treated in a similar manner to demonstrate tyrosine to L-dopa conversion. For casein, the conversion was monitored using the dopa-nitration reaction (Waite and Benedict, 1984, Methods in Enzymology, 107, 397-413). Parallel to casein, synthetic 5x decapeptide treated in Example 6 above was run as a

reaction control. Two proportions of enzyme to substrate, all in sodium phosphate buffer (0.1 M, pH 7.0) in the absence of L-ascorbic acid were monitored for 40 minutes, with the exception of the one assay which was monitored for 80 minutes. For synthetic decapeptide (5x repeat), the enzyme-to-substrate ratios were 13.5 and 21.6 Units/μ g. For casein, enzyme-to-substrate ratios were 1.3 and 5.1 Units/ μ g; for collagen, the ratios were 12 and 36 Units/ μ g. Reactions were in 1.0 ml sodium phosphate buffer (o.1 M, pH 7.0) at room temperature (21-23$^{O}$C). The data are shown in Figure 3. Tyrosines in all preparations are converted at similar rates. The same reactions were effected for collagen but samples could not removed for assay due to immediate gelation of the collagen by enzyme.

As described herein, L-ascorbic acid reverses the quinone formation, but it also competes in the crosslinking reaction. Where adhesive strength and cohesive strength is to be enhanced through covalent interactions, i.e. L-dopa crosslinks, L-ascorbic acid should be omitted from the reaction.

## Correlation of L-Dopa Conversion with Adhesivity

Adhesive strengths of the proteins were tested using aluminum strips as the bonding substrate, a one-square centimeter bond area and a vertical shear strength test (Table 3). These particular foil strips fail at about 600

grams. Conditions and ratios were varied as shown in Table 3 in order to clearly demonstrate the effect of tryosinase conversion on bond strength. Preincubations of casein and synthetic peptide with tyrosinase were performed to show the effect of increasing L-dopa content on bond strength. Bond strengths for the synthetic decapeptide increased with a higher enzyme ratio or with increased pre-incubation time at lower enzyme ratios. In the absence of L-ascorbic acid, a limit is reached between 5 and 10 minutes of pre-incubation time due to quinone formation. The same is true of between 10 and 20 minutes preincubation for casein.

TABLE 3

Adhesive Strengths Correlated With Tyrosine Conversion to L-dopa Residues Per 1,000 Residues

| Adhesive Protein | Tyrosinase (Units/μg) | Pre-incubation (minutes) | Breaking force ($gm/cm^2$) |
|---|---|---|---|
| 1. synthetic decapeptide | 0 | 0 | 0 |
| 5 x repeat | 21.6 | 0 | 300 |
| 40 μ g/bond | 13.5 | 0 | 0 |
| | 13.5 | 2 | 130 |
| | 13.5 | 5 | 175 |
| | 13.5 | 10 | 0 |
| 2. casein 40 μ g/bond | 13.5 | 0 | 540 |
| | 13.5 | 2 | 488 |
| | 13.5 | 5 | 650 |
| | 13.5 | 10 | 720 |
| | 13.5 | 20 | 290 |
| 3. collagen 45 μ g/bond | 12 | 0 | 600 |
| 30 μ g/bond | 36 | 0 | 600 |

Example 9

This example demonstrates the effect of the ratio of catechol oxidase to bioadhesive polyphenolic protein with a single pre-incubation time on bond strength. A constant amount of synthetic peptide, the linear decapeptide chain repeated five times, was incubated for 2 minutes with varying amounts of mushroom tyrosinse (catechol oxidase) prior to establishing the bond between two strips of aluminum foil. The bond dimensions are 1.3 $cm^2$. The

entire procedure is performed at room temperature. All bonds contain 40 μg of synthetic peptide in 1.0 μl of 0.1 M sodium phosphate buffer (pH 7.0). Mushroom tyrosinase concentrations were 5.4, 10.8, 21.6, 32.4, 43.2, and 54 Units per μg of synthetic peptide on the bond site. Volumes of liquid for each bond were kept constant at 5.0 μl with the addition of the appropriate amount of 0.1 M sodium phosphate buffer (pH 7.0). Each bond strength represents the average of five trials. Bonds were allowed to cure for one hour, then measured by clamping the strips between a pressure gauge (0-500 Gm range) and a geared motor with a piston producing strain at a rate of 25 grams per second. The data are shown in Figure 4. The fact that several variables may be manipulated is illustrated. With a set 2 minute pre-incubation time, 30-40 Units of enzyme per μg of bond protein is optimal. More enzyme can be tolerated if ascorbic acid is included. In this example, a parallel series with 0.5 μl of 0.1 M ascorbic acid in 0.1 M sodium phosphate buffer was included in the formulation. Both bond strength and rate of tyrosine to L-dopa coversion can be manipulated by any variable which affects the rate of this enzyme reaction. These include pH, temperature, and degree of use of oxidation and reducing agents. Beyond 40 units/μg protein, the enzyme protein concentration is itself contributing to bond strength as a dried protein cement.

WHAT IS CLAIMED IS:

1. Method of forming bioadhesive polyphenolic proteins containing 3,4-dihydroxyphenylalanine residues from protein precursors containing tyrosine residues comprising:

preparing a tyrosine-containing protein;

reacting said protein with a tyrosinase enzyme at a pH of from about 4.5 to about 8 and a temperature of from about 20° to about 37°C. at an enzyme to protein ratio of from about 5 to about 50 Units enzyme per μg protein; and thereby forming a bioadhesive polyphenolic protein containing 3,4-dihydroxyphenylalanine residues.

2. Method as defined in Claim 1 wherein the tyrosinase enzyme is mushroom tyrosinase.

3. Method as defined in Claim 1 wherein the reaction is conducted in the presence of sufficient ascorbic acid to effectively retard conversion of the 3,4-dihydroxyphenylalanine residues to quinones.

4. Method as defined in Claim 1 wherein the tyrosine-containing protein is obtained by solid phase peptide synthesis.

5. Method as defined in Claim 1 wherein the pH of the reaction ranges from 7 to 7.5.

6. Method as defined in Claim 1 wherein the reaction is conducted in a buffered medium.

7. Method as defined in Claim 1 wherein said tyrosine-containing protein and said enzyme are preincubated in admixture with each other for a period of time inversely proportionate to the enzyme-to-protein ratio.

8. Method as defined in Claim 7 wherein preincubation is effected for a period of from 0 to about 15 minutes.

9. Method as defined in Claim 1 wherein the reaction is terminated by decreasing the pH of the reaction system below about 4 and the bioadhesive polyphenolic protein containing 3,4-dihydroxyphenylalanine residues is recovered.

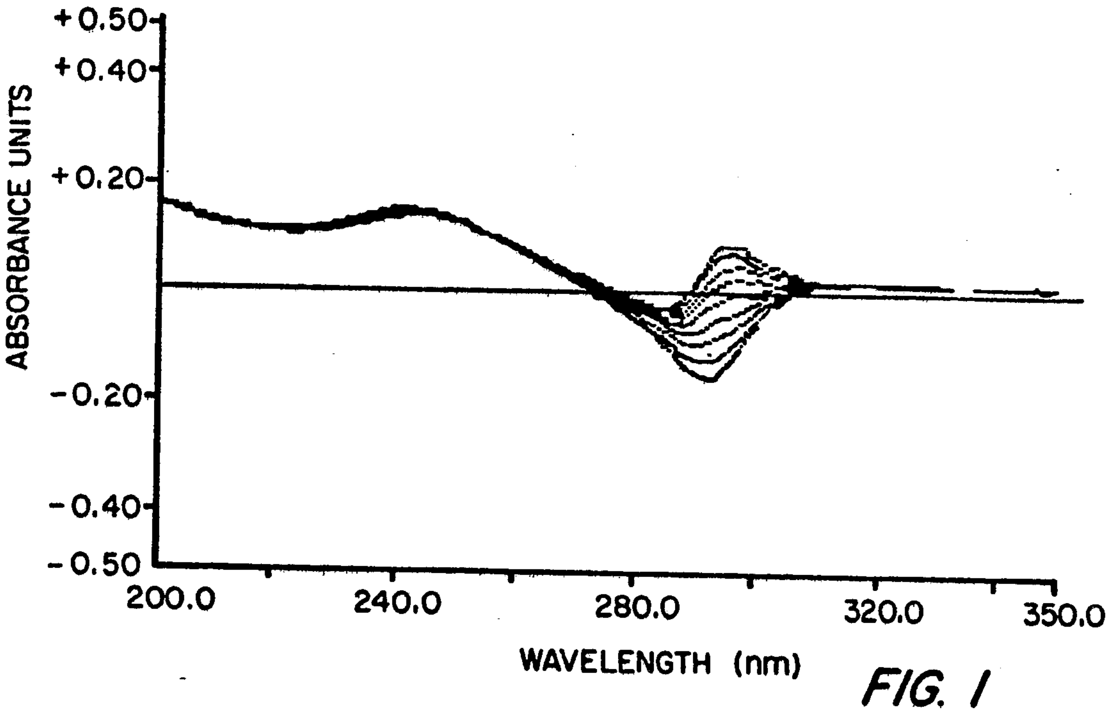


FIG. 1

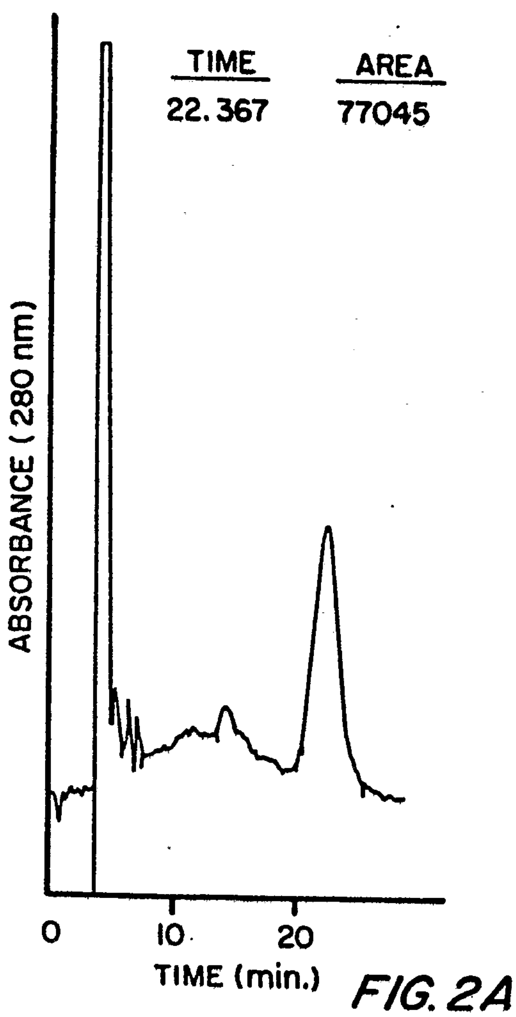


FIG. 2A

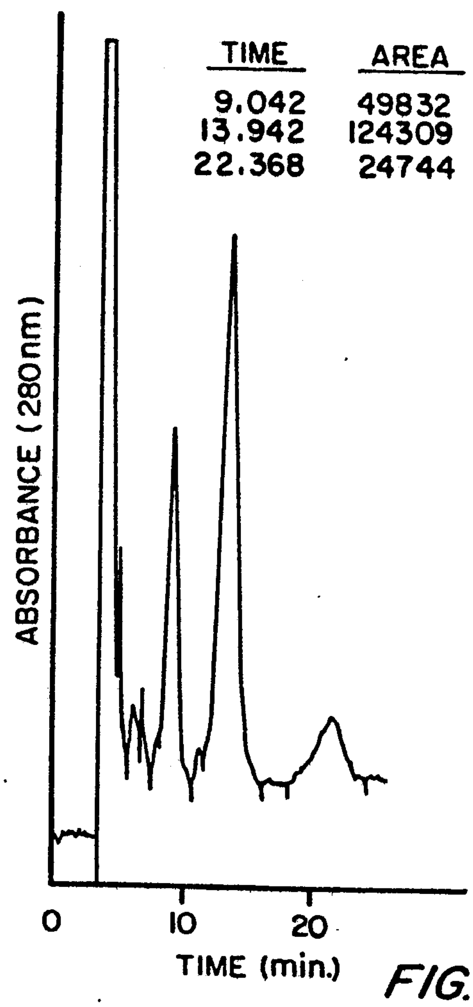


FIG. 2B

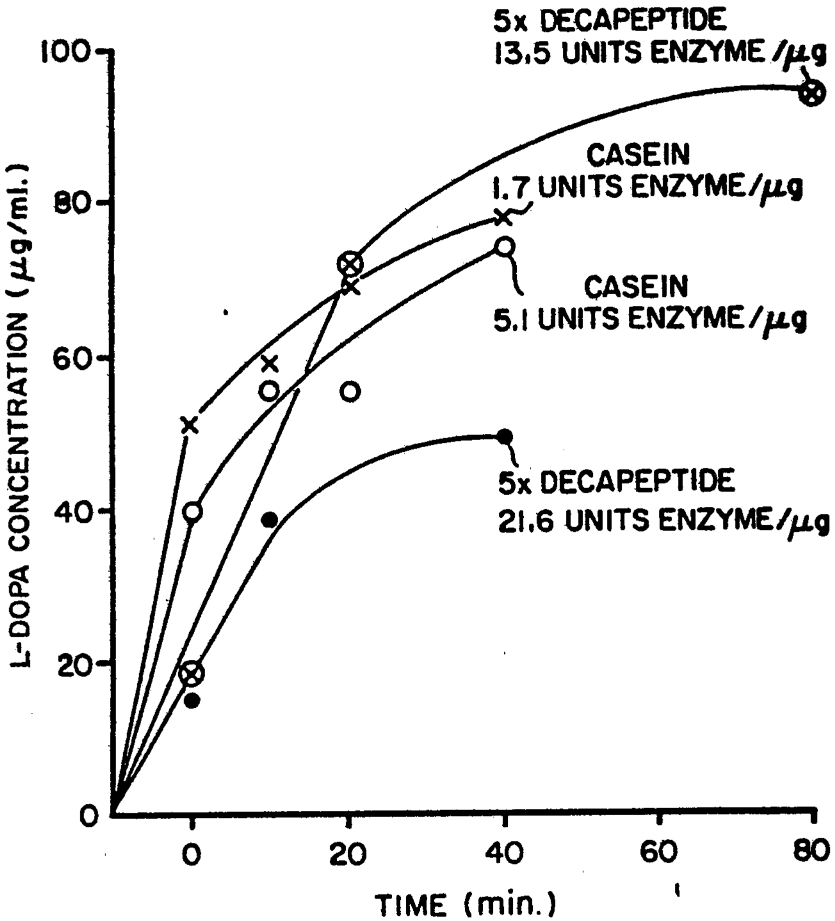
100
80
60
40
20
0
L-DOPA CONCENTRATION ( μg/ml.)
0
20
40
60
80
TIME (min.)
5x DECAPEPTIDE
13.5 UNITS ENZYME /μg
CASEIN
1.7 UNITS ENZYME/μg
CASEIN
5.1 UNITS ENZYME/μg
5x DECAPEPTIDE
21.6 UNITS ENZYME/μg

FIG. 3

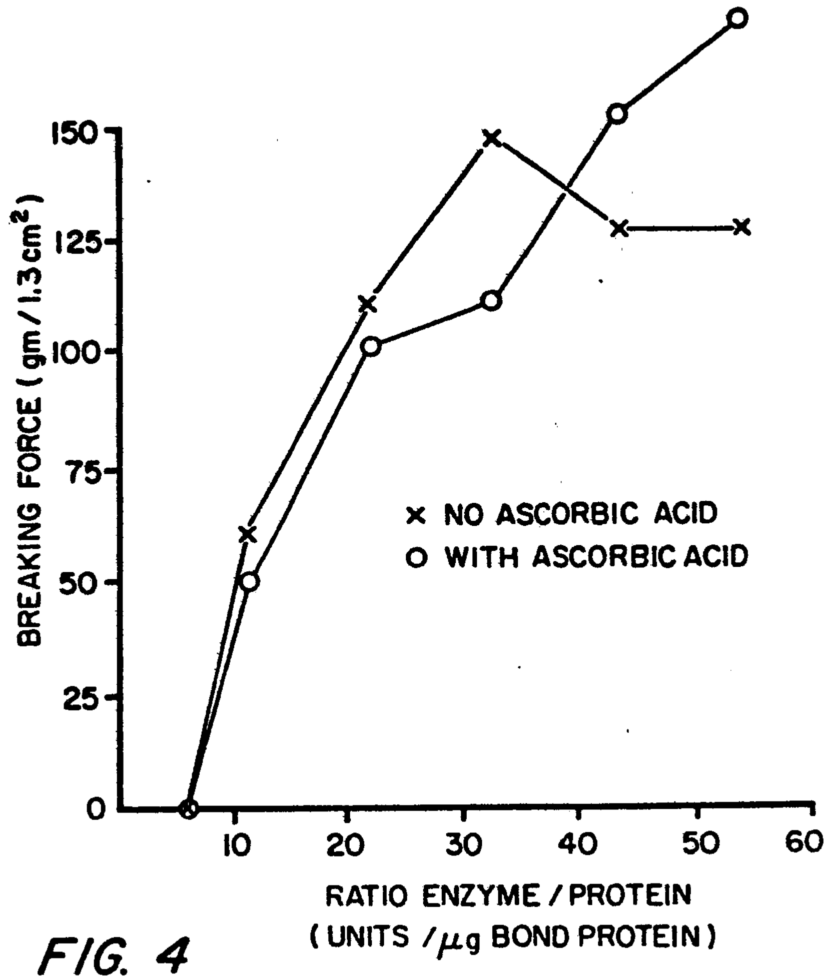
150
125
100
75
50
25
0
BREAKING FORCE ( gm / 1.3 cm²)
10
20
30
40
50
60
RATIO ENZYME / PROTEIN
( UNITS / μg BOND PROTEIN )
× NO ASCORBIC ACID
O WITH ASCORBIC ACID

FIG. 4